(19) Europäisches Patentamt
European Patent Office
Office européen des brevets

(11) **EP 1 839 705 A1**

(12) **EUROPEAN PATENT APPLICATION**

(43) Date of publication:
**03.10.2007 Bulletin 2007/40**

(51) Int Cl.:
***A61N 5/06*** *(2006.01)*

(21) Application number: **07380080.7**

(22) Date of filing: **26.03.2007**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK YU**

(30) Priority: **27.03.2006 ES 200600796**

(71) Applicant: **Universidad de Alcala**
**28801 Alcala de Henares (Madrid) (ES)**

(72) Inventors:
• **Montes Molina, Ramón**
**28801 Alcala de Henares (Madrid) (ES)**
• **Luque Lora, Javier**
**28801 Alcala de Henares (Madrid) (ES)**

(74) Representative: **Manzano Cantos, Gregorio**
**Cabinet Manzano**
**Embajadores, 55**
**28012 Madrid (ES)**

(54) **Transcutaneous laser therapy patch**

(57) The present invention relates to a low-intensity laser therapy patch consisting of a laser emitter for the transcutaneous and instant application, and which is designed with an output power of the laser diode that is fixed or variable, for selecting the laser energy density for the treatment of different medical pathologies.

*FIGURE 2*

**Description**

**FIELD OF THE ART**

The present invention relates to a low-intensity laser therapy apparatus for its transcutaneous and instant application in any part of the body for the treatment of different medical pathologies or aesthetic treatments.

**STATE OF THE ART**

Class IIIA, IIIB and IV low-intensity laser is applied therapeutically radiating both large body surfaces and point-based areas on small areas for the treatment of multiple diseases such as tendonitis, tendonosis, peripheral paralysis, muscle pains, epitrochleitis, epicondylitis, sprains, arthrosis of the base of the thumb, plantar heel pains, back pains, osteoporosis, contusions, herpes zoster, joint pains, muscle contractures, neuralgias, arthritis, sciatica, periarthritis, arthrosis, gonarthrosis, cervical arthrosis, edemas, fractures and injuries. These therapeutic applications are extended to different medical specialties: traumatology, rheumatology, dermatology, vascular, odontology and neurology (Jan Tuner and Lars Hode: Laser Therapy. Clinical practice and scientific background. Prima Books, Sweden, 2002)

The therapeutic effects of low-intensity laser are based on the biochemical, bioelectrical and bioenergetic effects caused by the photons in their interaction with biological structures. These effects are an increase of the production of adenosine triphosphate (ATP) and of proteins, an increase of cell proliferation and tropism, increase in microcirculation, changes in the permeability of the cell membrane, increase in endorphin and serotonin levels, increase of lymphocytes and increase of lymphatic flow.

The traditional technique of applying low-intensity laser therapy is carried out by means of equipment located in hospitals and clinics for a certain number of sessions and by means of short time periods. The large limitation existing in the clinical application of this technique consists of the existence of a time period from its clinical indication until its therapeutic application on the patient, and this technique not being available immediately or in the patient's home.

The laser energy penetrates more deeply in the skin contact technique; it is applied by means of sensors that the therapist applies manually and requires more treatment time.

The non-contact technique uses a higher density of laser power to compensate for the distance between the opening of the beam and the treatment area, effective treatment times are shorter and they usually automatically radiate the beam by scanning it over the tissue.

In both techniques, the action depth of the low-intensity laser therapy, combining the direct and indirect effects, can reach up to 3 cm and is in accordance with the wavelength, intensity and collimation of the beam and of the anatomical feature of the application surface.

The different parameters forming part of the correct selection of the dosage of low-intensity laser therapy are: the rated power of the laser (milliwatts, mW), laser power density (watts per square centimeter, $W/cm^2$), energy (Joules, J), energy density (Joules per square centimeter, $J/cm^2$), treatment surface area (square centimeter, $cm^2$), distance from the laser emission point to the therapeutic target (centimeters, cm), radiation time, type of the emission: pulsating or continuous, working cycle (%), pulse repetition frequency (cycles per second or Hertz, Hz), pulse duration (nanoseconds or microseconds, ns or ms), laser wavelength (nanometer, nm) and beam divergence (degrees, °).

The dosage of the low-intensity laser energy is comparable to a pharmacological model in which a substance is supplied at a certain dose for the purpose of achieving a therapeutic effect.

The most widely used parameter for the laser therapy dosage is the energy density, which is quantified in $Joules/cm^2$ and uses the following formula for its calculation:

$$J/cm^2 = \frac{\text{Average power (W) x Exposition time (s)}}{\text{Surface area } (cm^2)}$$

There is a laser energy dosage for the treatment of different pathologies according to the previously mentioned parameters based on the therapeutic experience of years of using this technique. For example, the following are the energy density dose for lower back pains (6 $J/cm^2$), periarthritis (6 $J/cm^2$), gonarthrosis (4 $J/cm^2$), epicondylitis (3 $J/cm^2$), sprain (4 $J/cm^2$), carpal tunnel syndrome (4 $J/cm^2$). The dosage based on energy density generally ranges from 1 $J/cm^2$

to 99 J/cm$^2$.

Laser therapy equipment today is carried out in solid state or by means of semiconductor laser diodes. High-intensity light emitting diodes (LED) have reduced this technology, which have allowed the use of radiation with a diagnostic target of the laser in small or point-based areas such as in Doppler laser equipment, laser oximetry or laser spectroscopy.

The permanent or transient application of different types of therapeutic patches is used in the treatment of different diseases, for example: patches with medicinal products (US 2004097864), (US 6143320), (US 5154182), (US 2004043062), (JP 2002121136), (HV 0002085); electrostimulation patches; iontophoresis patches (MXP A01007996), (US 2002183685), (KR 2002013249), (US 6424862); heat-generating patches (US 2004097864); magnetotherapy patches (US 6344021), (US 3943912), (US 4587956), (US 5312321), (US 5707333). However, there are no therapeutic low-intensity laser patches.

**EXPLANATION OF THE INVENTION**

The present invention relates to a laser therapy patch for its transcutaneous application, applied instantly, having automatic operation and being used at home.

The laser therapy patch consists of a power supply or battery, a control circuit supervising functioning and activating the laser diode which generates a visible light or infrared laser beam.

The dose is administered instantly and automatically by means of the patient's activating an "on" button and the deactivation by an "off" button (although both functions can be carried out by a single "on/off" button according to the prior art and for the purpose of simplifying and reducing space).

The laser patch is placed transcutaneously and all the parameters forming the laser energy dosage are kept constant with the exception of the exposure time, which is variable, whereby a different energy density is generated for each disease.

With the laser patch, the laser energy dosage applied in each treatment according to its pathology is based on the fact that the parameters for calculating the energy density (J/cm$^2$), mean power (W), energy (J) and the treatment surface area (cm$^2$) remain steady, changing the exposure time according to the energy dose (J) indicated for each treatment.

Based on the foregoing, a different energy density is obtained for each clinical situation according to the following formula:

$$\text{Time} = \frac{\text{Dose per Surface}}{\text{Mean power}}$$

The mean power is calculated as follows:

$$\text{Mean power (Wm)} = \text{Wp} \times \text{Tp} \times \text{F}$$

Where Wm is the mean power (W), Wp is the peak power (W), Tp is the pulse duration (ns or ms) and F is the pulse frequency (Hz).

In the most simplified type, the considered power will be the maximum power of the laser diode given that a pulse working regimen is not applied, its functioning being continuous and therefore the mean power will be equal to the maximum power of the laser diode.

$$Time\ (s)\ =\ \frac{Energy\ (J)\ x\ Surface\ (cm^2)}{Mean\ power\ (W)}$$

The advantages of this type of application of laser therapy are: ability to be reused by the patient for an undetermined number of sessions (the battery can be replaced), local therapeutic actions, non-invasive and painless technique, transcutaneous placement, immediate and instant application, non-pharmacological therapeutic agents, offering multiple pathologies for treatment, portable application (it is not necessary to go to a clinic or hospital to apply it), technique that is complementary or alternative to traditional therapy, very simple activation and automatic operation, low consumption and low cost. Since the laser is located in the part in contact with the skin and the upper part is made of an opaque material, it is not necessary to wear glasses for its therapeutic use.

**DESCRIPTION OF THE DRAWINGS**

Figure 1 shows a circuit block diagram of the transcutaneous laser therapy patch. It shows the battery or power supply (1), on-off position switches (2 and 3), control circuit (7), operating indicator (4), low battery indicator (5) and laser emitter (6).

Figure 2 shows an upper view of the laser therapy patch, showing the "on" button (8) for the activation of the laser once positioned in the place to treat, the "off" button (12) for deactivating the laser, the operating indicator (9) notifying that the laser is on, the low battery indicator (11) notifying that the battery needs to be replaced and a battery cover (10) for accessing the battery compartment to replace it.

Figure 3 shows a lower view of the laser therapy patch, showing the laser output opening (14) and adhesive (15).

Figure 4 shows a cross-section view of the laser therapy patch, showing the "on/off" buttons (16), operating and low battery indicators (18), the battery (17), adhesive (22), a diode or laser diode array or laser emitter (21) including the optics for distributing the beam, an opening (20) where the laser diode beam exits and a control circuit (19) for controlling and exciting the laser diode.

Figure 5 shows a replaceable adhesive system for holding the laser therapy patch to the surface of the body with its central part (23) and peripheral part (24).

**EMBODIMENT**

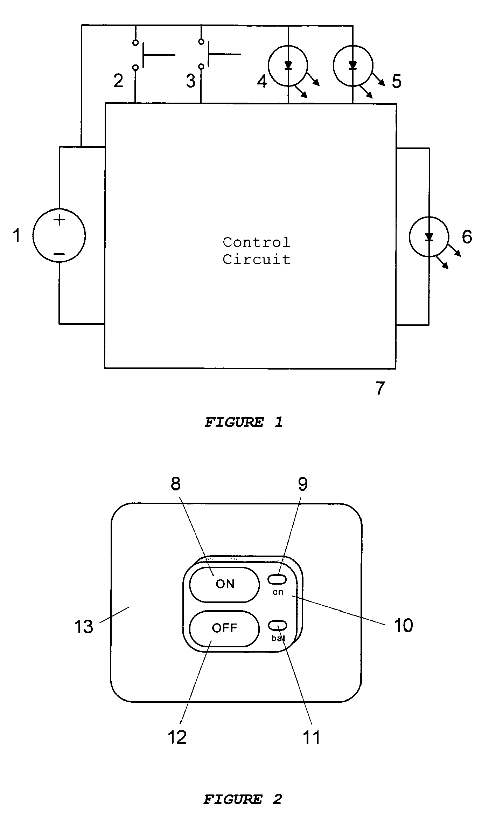

The laser therapy patch (Figure 1) consists of a battery or power supply (1 and 18), a control circuit (2 and 19), diode or diode array or laser emitter (6 and 21) and a changeable adhesive system (13 and 15).

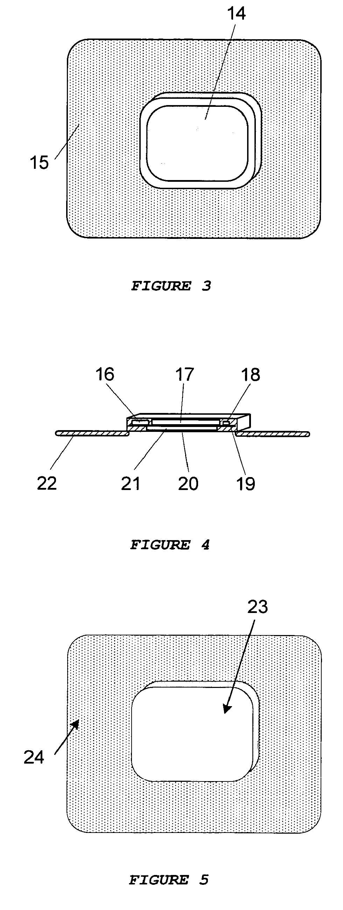

The optical system is located in the central lower part (Figure 3) and is formed by an opening (14 and 20) which may have a lens and a laser diode or diode array.

The laser diode or diode array generates a visible light or infrared laser beam. The output power of the laser diode or diode array will depend of the area to be treated and will therefore be treated according to its class. The laser diode or diode array will be excited from the control circuit.

The control circuit continuously supervises the correct operation of each of the components to assure the output power of the laser diode and for prevent overheating or malfunction. The energy for feeding the control circuit and therefore the laser diode or diode array is powered by the battery although a feed input can be provided from an external feeder or power supply.

The lower part of the patch (Figure 3) contains an opening (14) in its central part from where the laser radiation is emitted. The dimensions of said opening will be suitable for the surface area to be treated.

The "on" and "off" buttons (16) (or just one "on/off" button for simplifying and reducing space) are located in the upper part of the laser patch (Figure 4) to control the start of treatment and for the interruption thereof, and operating and low battery indicators (17).

An adhesive system (Figure 5) allows maintaining the patch on the surface of the body and consists of a central dome part (23) covering the patch of a transparent and flexible material and an adhesive peripheral part (24) coated with a coating paper.

The present invention is additionally illustrated by means of the following example of the treatment parameters, which do not aim to be limiting of its scope.

Example: Treatment of Epicondylitis

Therapeutic target: To administer an energy density of 2 J/cm$^2$.
According to formula:

$$\text{Time} = \frac{2 \text{ J/cm}^2 \times 2.25 \text{ cm}^2}{5 \times 10^{-3} \text{ W}} = 900 \text{ s} = 15 \text{ min}$$

Energy density (2 J/cm$^2$)
Power of the diode (5 mW)
Treatment surface (2.25 cm$^2$)
Radiation time = 15 minutes

**INDUSTRIAL APPLICATION**

The invention can be applied in the electromedicine, physiotherapy, rehabilitation, aesthetics, odontology and veterinary fields.

**Claims**

1. A transcutaneous laser therapy patch **characterized in that** it consists of a power supply or battery (1) activating an operating control circuit (7 and 19) exciting a semiconductor diode or diode array (6 and 21) or another laser emitter for its instant transcutaneous application with automatic functioning by means of an independent adhesive system (13, 15, 23 and 24).

2. A transcutaneous laser therapy patch according to claim 1, **characterized in that** the laser used is a class IIIA or IIIB low-intensity laser and can be formed by one or several laser diodes or diode array, or other laser emitters (6 and 21) acting simultaneously or sequentially and with continuous or pulsating emission in any of its combinations.

3. A transcutaneous laser therapy patch, according to claims 1 and 2, **characterized in that** it contains a control circuit powered by a battery or power supply (1 and 18), exciting the laser emitter and supervising and controlling the parameters of the laser energy to be applied in different therapeutic treatments. The control circuit supervises the power and prevents the overheating or malfunction of the system.

4. A transcutaneous laser therapy patch, according to claims 1 and 3, **characterized in that** the control system consists of an activation and deactivation button for starting and ending the treatment (2, 3, 4, 8, 9, 12 and 16) as well as the operating state of the battery or power supply (5 and 11).

5. A transcutaneous laser therapy patch according to the previous claims, **characterized in that** its external configuration can vary in design, shape and size, as well as the configuration of the laser equipment and the skin adherence system according to the surface areas to be treated.

6. A transcutaneous laser therapy patch according to the previous claims, **characterized in that** it has an industrial application in the electromedicine, physiotherapy, rehabilitation, aesthetic, odontology and veterinary fields, with advantages of use by the patient immediately and instantly, non-invasive techniques and easy to use and operate.

**FIGURE 1**

**FIGURE 2**

14

15

FIGURE 3

16    17    18

22    21    20    19

FIGURE 4

23

24

FIGURE 5

**EP 1 839 705 A1**

European Patent
Office

**EUROPEAN SEARCH REPORT**

Application Number

EP 07 38 0080

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DE 101 28 629 A1 (NOVICUR AG BALZERS [LI]) 19 December 2002 (2002-12-19) * paragraph [0041] - paragraph [0044]; figures 1,2 * | 1-6 | INV. A61N5/06 |
| X | US 2004/220513 A1 (STREETER JACKSON [US]) 4 November 2004 (2004-11-04) * paragraph [0015] - paragraph [0016]; figures 1,2 * | 1-6 | |
| X | US 5 616 140 A (PRESCOTT MARVIN A [US]) 1 April 1997 (1997-04-01) * figures 1a,2 * * column 4, line 55 - column 5, line 10 * * column 5, line 62 - column 6, line 65 * * column 11, line 63 - column 12, line 4 * | 1-6 | |
| P,X | WO 2006/107387 A (ADVANCED PHOTODYNAMIC TECHNOLO [US]) 12 October 2006 (2006-10-12) * page 6, line 1 - line 13; figure 1 * | 1-6 | |
| X | DE 10 2004 018340 A1 (TEICHERT KLAUS [DE]) 10 November 2005 (2005-11-10) * paragraphs [0031], [0042] * | 1-6 | TECHNICAL FIELDS SEARCHED (IPC) A61N |
| X | WO 00/15296 A1 (LIGHT SCIENCES LP [US] LIGHT SCIENCES LTD PARTNERSHIP [US]) 23 March 2000 (2000-03-23) * page 8, line 4 - line 28 * | 1-6 | |
| X | US 2005/237739 A1 (LEE KIAN S [MY] ET AL LEE KIAN SHIN [MY] ET AL) 27 October 2005 (2005-10-27) * paragraph [0043] * | 1-6 | |
| X | US 2005/187597 A1 (VANDERSCHUIT CARL R [US]) 25 August 2005 (2005-08-25) * paragraph [0042] * | 1-6 | |

The present search report has been drawn up for all claims

| Place of search | Date of completion of the search | Examiner |
|---|---|---|
| The Hague | 24 July 2007 | Petter, Erwin |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 07 38 0080

This annex lists the patent family members relating to the patent documents cited in the above-mentioned European search report.
The members are as contained in the European Patent Office EDP file on
The European Patent Office is in no way liable for these particulars which are merely given for the purpose of information.

24-07-2007

| Patent document cited in search report | | Publication date | Patent family member(s) | | Publication date |
|---|---|---|---|---|---|
| DE 10128629 | A1 | 19-12-2002 | WO | 02100484 A1 | 19-12-2002 |
| US 2004220513 | A1 | 04-11-2004 | NONE | | |
| US 5616140 | A | 01-04-1997 | AU | 2104195 A | 09-10-1995 |
| | | | WO | 9525563 A1 | 28-09-1995 |
| WO 2006107387 | A | 12-10-2006 | US | 2006173514 A1 | 03-08-2006 |
| DE 102004018340 | A1 | 10-11-2005 | NONE | | |
| WO 0015296 | A1 | 23-03-2000 | AU | 750568 B2 | 25-07-2002 |
| | | | AU | 5575799 A | 03-04-2000 |
| | | | CA | 2341235 A1 | 23-03-2000 |
| | | | EP | 1109599 A1 | 27-06-2001 |
| | | | JP | 2003526391 T | 09-09-2003 |
| | | | US | 6096066 A | 01-08-2000 |
| US 2005237739 | A1 | 27-10-2005 | NONE | | |
| US 2005187597 | A1 | 25-08-2005 | US | 2006235494 A1 | 19-10-2006 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2004097864 A
- US 6143320 A
- US 5154182 A
- US 2004043062 A
- JP 2002121136 B
- US 2002183685 A
- KR 2002013249
- US 6424862 B
- US 6344021 B
- US 3943912 A
- US 4587956 A
- US 5312321 A
- US 5707333 A

**Non-patent literature cited in the description**

- **JAN TUNER ; LARS HODE.** Laser Therapy. Clinical practice and scientific background. Prima Books, 2002